# EUROPEAN PATENT SPECIFICATION

(11) **EP 0 809 986 B1**
(45) Date of publication and mention of the grant of the patent: **16.10.2002**
(21) Application number: 97303470.5
(22) Date of filing: 21.05.1997
(51) Int. Cl.: A61F 2/38

(54) **Tibial element for a replacement knee prosthesis**
Tibiateil für eine Knieprothese
Elément tibial pour prothèse de genou

(30) Priority: 28.05.1996 GB 9611059
(43) Date of publication of application: 03.12.1997
(60) Divisional application: 01124753.3
(73) Proprietor: HOWMEDICA INTERNATIONAL S. DE R.L., Shannon Co. Clare (IE)
(72) Inventor: Ashby, Alan Miles, Lymington, Hampshire S041 OTX (GB); Dorrell, Paul Frank, Castleconnell, County Limerick (IE)
(74) Representative: Bridge-Butler, Alan James

(56) References cited:
- EP-A- 0 552 950
- EP-A- 0 592 750
- EP-A- 0 634 155
- EP-A- 0 634 156
- EP-A- 0 678 286
- US-A- 4 711 639

## Description

This invention relates to a tibial element for a replacement knee prosthesis of the kind comprising a tibial tray provided with one or more bearing components.

It is known to provide a tibial tray with an attachment element, for example a stem, which can be selected and fitted by the operating surgeon. Thus, trays can be provided with a variety of stems of different lengths and construction which can be used in accordance with the surgical and medical requirements at the time the prosthesis is fitted.

European Patent Application No. 0 678 286 shows a tibial tray construction which includes a boss on its lower side. An attachment element is secured to the boss by a retaining bolt. The upper surface of the tray carries a rotatable bearing component which is located by an upwardly projecting ring about which it can rotate. In one construction the bearing component is designed for use when the cruciate ligament is removed from the knee and thus carries an upwardly projecting cam. The connecting bolt has an upward extension which extends into the cam to reinforce it to enable it to resist the forces generated by the cam follower on the femoral component which engages the cam but the bearing component is held in place on the tibial tray by the femoral component itself. There is no securing means between the bearing component and the tibial tray.

In the construction shown in the Applicants' European Application No. 0 552 950 the stem is held to the tray by means of a tapered spigot and socket connection which is locked in place by a fixing screw 60. The bearing component is held in position by a capture system employing a number of small catches. This capture arrangement entails a number of accurately made features and the present invention is intended to provide a simplified construction which will not only be more effective but easier to manufacture.

According to the present invention a tibial element for a replacement knee prosthesis comprises a tibial tray, fastening means for securing an attachment element to the lower part of said tray, characterised in that the upper end of said fastening means is also provided with independently operable securing means which engage a co-operating construction on a bearing component to secure the bearing component to the upper part of the tibial tray.

Thus, with the present arrangement the fastening means allows for a stronger construction than the previous arrangements and it also allows for a convenient modular construction which will allow a standard modular tray to be employed with various types of bearing construction, for example a fixed component or a sliding component.

Preferably the independently operable securing means are releasable thus allowing the bearing component to be removed and replaced without disturbing the connection of the tibial element to the bone.

As mentioned above the bearing component can be arranged to slide in relation to the tray when secured or alternatively it can be fixed in position in relation to the tray.

The fastening means can be provided with a boss at its upper end which is shaped to co-operate with the co-operating retaining construction on the bearing component.

Such a boss can have a projecting flange shaped to engage a securing flange provided on the bearing component.

Two bearing components can be provided each component having a co-operating construction which engages the independently operable securing means and if desired each bearing component can be independently engageable with the securing means.

In a preferred construction the bearing component is a snap fit onto the independently operable securing means provided on the fastening means to relatively secure said bearing component.

The invention is intended to provide good post operative stability of the joint, provide a low sensitivity to surgical technique and soft tissue quality and provide mechanisms to avoid the likelihood of insert dislocation and other bearing damage.

The tray can be standard for both left and right knees and the bearing components handed. With this arrangement the tray can be substantially symmetrical about a vertical axis.

The invention can be performed in many ways but some embodiments will now be described by way of example and with reference to the accompanying drawings in which :
Figure 1 is a part cross-sectional side elevation of a tibial element according to the present invention;
Figure 2 is an exploded view of the construction shown in Figure 1 with the bearing component removed;
Figure 3 is a cross-sectional side elevation of the bearing component shown in Figure 1;
Figure 4 is a plan view from below of the bearing component shown in Figure 3;
Figure 5 is a diagrammatic representation showing how the bearing component is placed in position on the tibial tray;
Figure 6 is a part cross-sectional side view showing the bearing component in the position shown in Figure 5 and about to be pushed into position;
Figures 7 and 8 are views similar to Figure 6 showing the bearing component in sequential loading positions of assembly;
Figure 9 is a view similar to Figure 6 showing the bearing component after loading and in an anterior position;
Figure 10 is a view similar to Figure 9 showing the bearing component after loading and in a posterior position;
Figure 11 is a diagrammatic plan view illustrating the range of movement of the bearing component on the tray;
Figure 12 is a view similar to Figure 9 but showing a construction in which the bearing component is not removable;
Figure 13 is a diagrammatic plan view of the bearing component in position on the tibial tray and showing additional features which can be incorporated;
Figure 14 is a view similar to Figure 9 showing another alternative construction in which the bearing component is fixed in relation to the tray;
Figure 15 is a view similar to Figure 4 showing a tray for use with the bearing component shown in Figure 14;
Figure 16 is a view similar to Figure 9 showing another alternative construction in which the position of the bearing component is fixed;
Figure 17 is a cross-sectional view through the boss shaped head of the screw which retains the stem and for use in the construction shown in Figure 16;
Figure 18 shows an alternative construction using two bearing components; and,
Figure 19 shows another alternative construction for use with two bearing components.

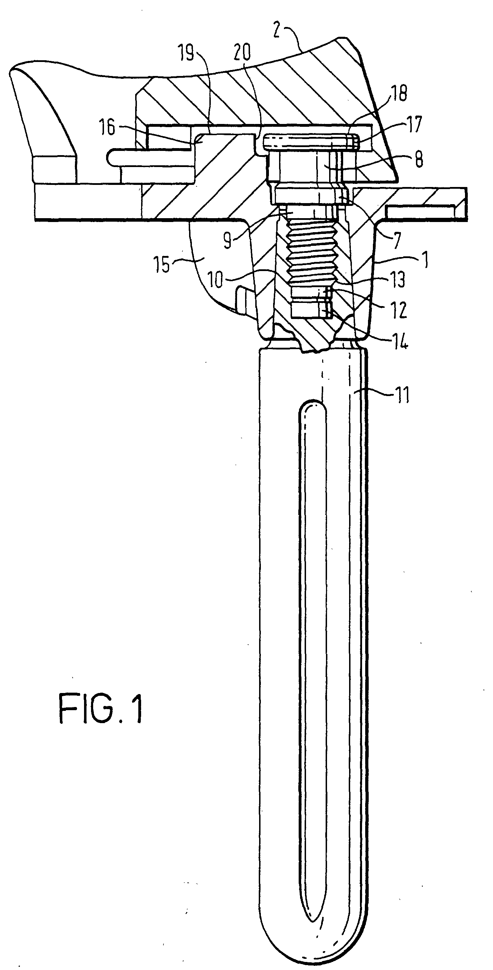

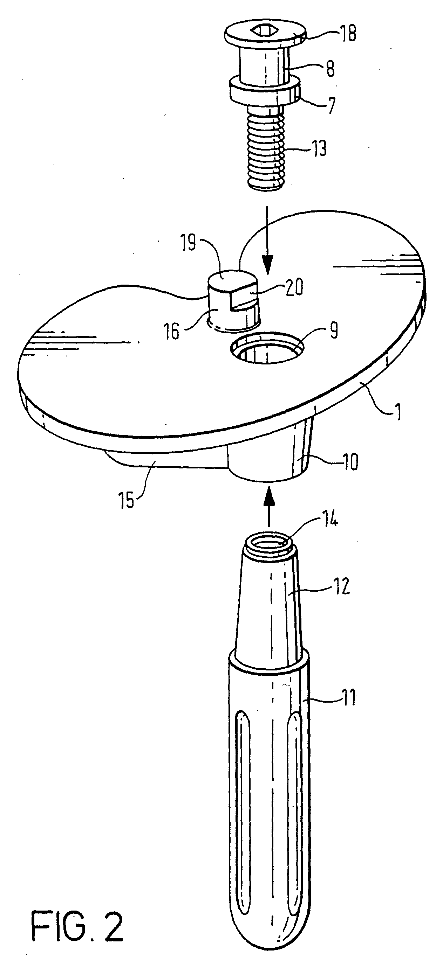

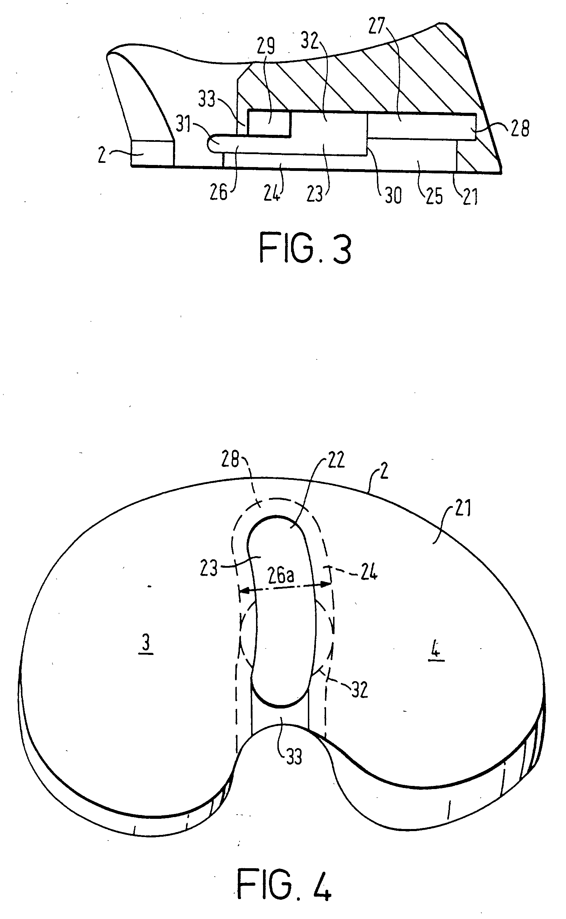

As shown in Figures 1 and 2 a tibial element for a replacement knee prosthesis comprises a tibial tray 1 on which is carried a bearing component 2 having medial and lateral compartments respectively 3 and 4 and which are best shown in Figure 4. The upper surfaces of the compartments 3 and 4 are shaped to provide bearing surfaces 5 and 6. The tray itself is standard for both left and right knees and is substantially symmetrical about a vertical axis.

Fastening means are provided which act to secure an attachment element in the form of a stem to the lower part of the tray. These are in the form of a screw 7 having an enlarged boss shaped head 8. The lower part of the boss bears against a flange 9 on the tray and enters a tapered opening 10 where it acts to retain a stem 11 which has a co-operating tapered spigot 12 by engaging a screw thread 13 in a socket 14.

The lower surface of the tray can be provided with shaped engagement features 15 intended for engagement with the proximal sub-condylar area of the tibia of the patient and the general construction of the connection between the tray and the stem can, for example, be as set forth in the Applicants' European Patent Application **0 552 950** (H.42).

The upper surface of the tray 1 is provided with a control abutment 16 the function of which will be defined hereunder. It will be seen that the boss 8 of the screw 7 projects upwardly and the upper end is provided with a flange 17. When in position the upper surface 18 of the boss 8 is substantially horizontally in line with the upper surface 19 of the abutment 16. As is most clearly shown in Figures 1 and 2 the side of the abutment 16 adjacent the boss 8 is cut away to provide a recess 20 within which the flange 17 of the boss 8 is located when the screw 7 is in position.

As shown in Figures 3 and 4 the bearing component 2 has medial 3 and lateral 4 compartments and can be made from any suitable bearing material, for example ultra high molecular weight polyethylene. The lower surface 21 of the bearing component 2 is shaped to provide a curved track 22 which is most clearly shown in Figure 4. This curved track 22 is provided by a recess 23 which is formed with a peripheral inwardly protruding securing flange 24 around its edges. At the anterior end of the recess 23 the flange is deeper and is indicated by reference numeral 25.

Above the flange 24 the recess 23 is shaped to provide two horizontally extending grooves, the lower groove being indicated by reference numeral 26 and an upper groove 27 above the deeper portion 25 of the flange 24. The anterior end of the upper groove 27 is in the form of a radiused portion 28 so that the groove is closed at this end. The posterior end of the upper groove also has a radiused portion 29.

The posterior ends 31 of the lower groove 26 are open and emerge out of the side wall of the bearing component 2.

At the point where the lower posterior groove 26 meets the upper anterior groove 27 there is an enlargement provided by a circular vertically extending well 32, the diameter of which is equal to the horizontal distance extending between the base of the groove on either side of the recess 23.

The posterior end of the upper groove 27 is closed by a wall 33.

The horizontal distance between the base of the grooves 26 and 27 and indicated by arrows 26a is slightly more than the diameter of the flange 17 on the boss 8, the vertical depth of the lower posterior groove 26 is slightly greater than the vertical thickness of the flange 17 and the vertical depth of the upper anterior groove 27 is slightly greater again.

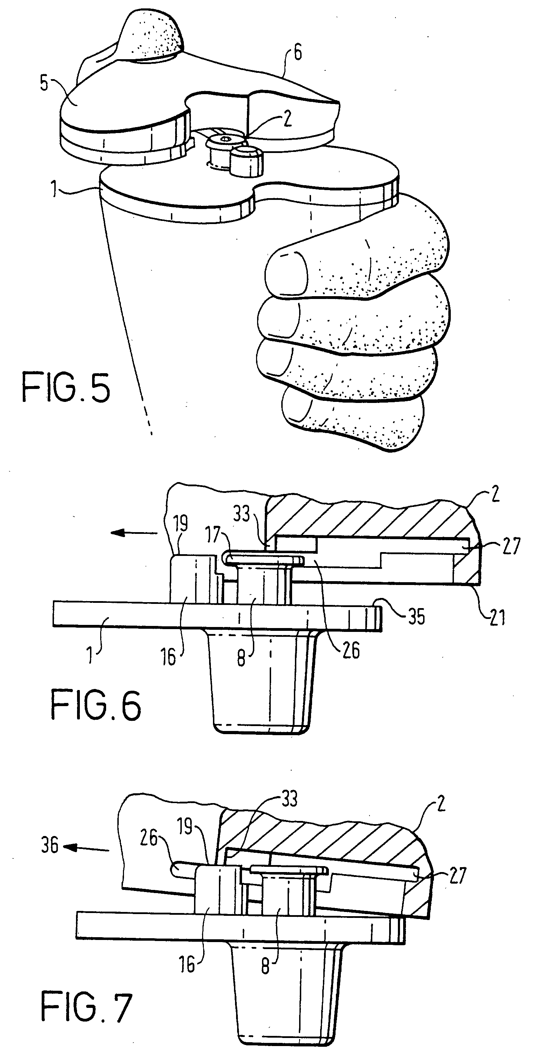

Figures 5 to 10 show how the bearing component 2 is placed in position and located on the tibial tray 1.

In the position shown in Figures 5 and 6 the bearing component is inserted by pushing its posterior side towards the boss 8. At this position, as shown in Figure 6, the lower surface 21 of the bearing component is raised above the upper surface 35 of the tray so that the open ends 31 of the lower posterior groove 26 engage over the flange 17 of the boss 8.

Further movement in the direction of the arrow 36 in Figure 7 shows that the bearing component 2 now has to be tipped to allow the flange 17 on the boss 8 to pass upwardly through the well 32 and into the upper anterior groove 27. The tipping movement is caused due to the upper surface 19 of the abutment 16 engaging the wall 33 at the end of the upper groove 28, and the flange 17 passing upwards in the well 32.

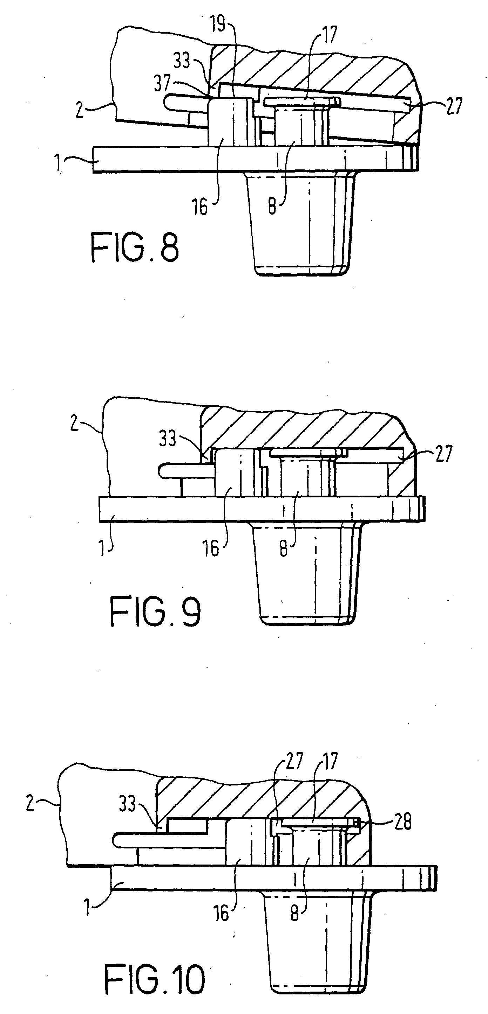

Further posterior movement achieves the position shown in Figure 8 in which the anterior side of the flange 17 of the boss 8 has entered the upper groove 27 but the posterior corner 37 is still engaging the lower corner of the wall 33.

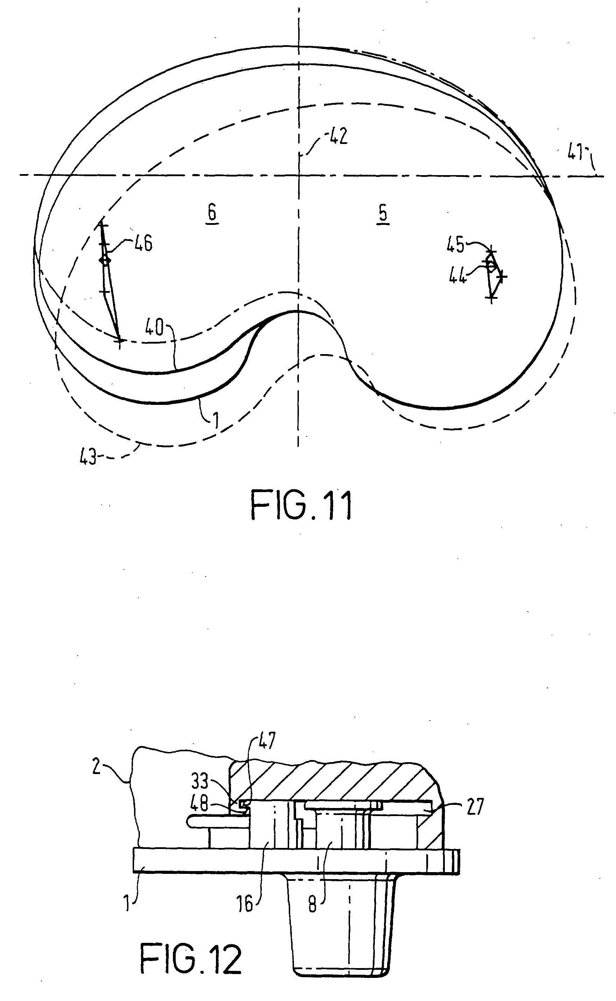

Due to the resilient nature of the material from which the bearing component is made, UHMWPE, the bearing component can now be snapped downwards by resiliently deforming the end of the wall 3 over the corner 37 of the abutment 16 to the position shown in Figure 9 where the bearing component is in its most anterior position. It will be seen that the abutment 16 now engages within the curved portion 29 at the posterior end of the upper groove 27 and the wall 33 prevents further anterior movement.

The bearing component can however move in a posterior direction until the boss 8 engages the anterior end of the recess 23 as shown in Figure 10. The flange 17 acting in the upper groove 27 prevents vertical removal of the bearing component and its horizontal movement on the tray 1 is controlled by the control abutment 16 and boss 8 which are located in tandem in the curved track 22, and act as guide means.

Thus the boss 8 provides a guide and with its flange 17 provides independently operable means for securing the bearing to the tray, the bearing component being a resilient snap fit into the guide which can be releasable.

The abutment 16 and boss 8 which provide the guide means and which are in tandem together act as control means between the tray and the bearing component to allow free posterior and anterior movement of the lateral compartment 4 which is greater than the small amount of free posterior and anterior movement of the medial compartment 3 in relation to the tray 1.

Figure 11 shows the relative movement. The central position of the bearing component 2 on the tray 1 is indicated by solid line 40. The general axes of the tray 1 are indicated by broken lines 41 and 42. From these it will be seen that in plan view the tray 1 is symmetrical about the centre line 42 but the medial compartment 5 of the bearing component 2 is larger than the tibial compartment 6.

From this central position the maximum posterior movement of the bearing component is indicated by broken line 43 and it will be seen that the tibial compartment has rotated about a mobile axis 44 the locus of the movement of which is indicated by the lines and crosses 45. The locus of movement of a similar point on the lateral compartment 6 is indicated by crosses and lines 46 and the much greater range of movement will be apparent.

If desired the shape of the track 22 can be arranged so that there is virtually no relative free posterior movement and anterior movement of the medial compartment 5.

In the construction described and shown in the drawings the control means acting between the tray 1 and the bearing compartment 2 allow rotational movement of the lateral compartment 6 in relation to the tray 1 about the pivotal axis 44 centred on the medial compartment and the arrangement allows restricted anterior and posterior movement of this pivotal axis.

It will be appreciated that other means for controlling the movement of the lateral compartment could be employed, for example, the control means could be in the form of a pivot which provides an axis of rotation and which would be centred on the medial compartment. Such a pivot could even allow a restricted free posterior and anterior movement relative to the tray.

Figure 12 shows an alternative construction in which the bearing component 2 is not removable once it has been fitted. In this construction the abutment 16 is provided with a projecting lip 47 which can engage a co-operating lip 48 on the wall 33 to prevent the bearing component 2 from being tipped to allow the wall 33 to be resiliently deformed and sprung over the corner 37 of the abutment 16.

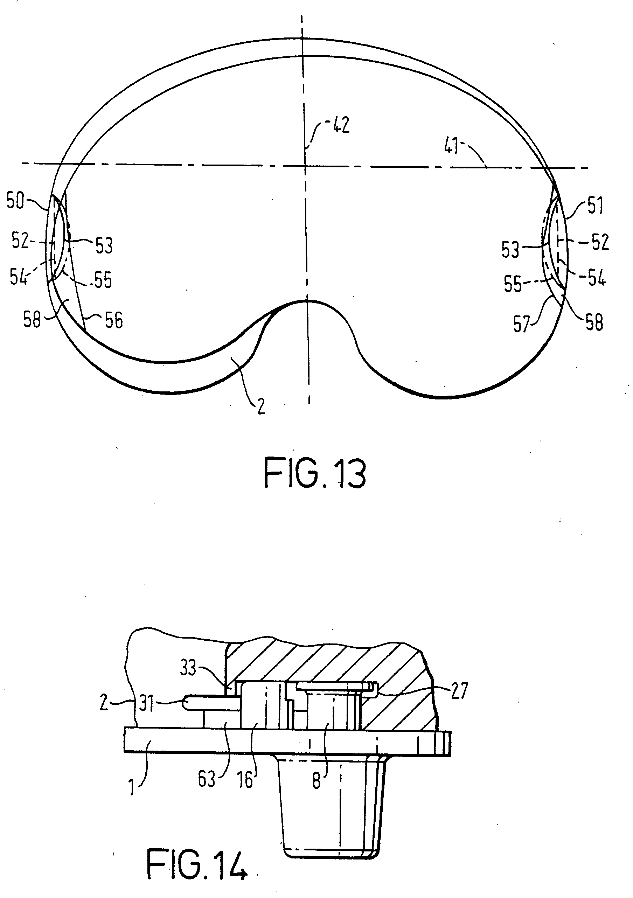

Figure 13 shows a construction in which a multi-functional tibial tray is employed and which can be used for a construction as described above and as shown in the drawings or with one in which the bearing component is fixed in position in relation to the tray 1.

With this construction medial and lateral retaining means are provided in the form of abutments 50 and 51. Each abutment comprises an upwardly projecting portion 52 and a horizontally projecting flange 53. The bearing component for use in this construction has an outwardly projecting flange 54 enclosed in a cut out segment 55 indicated by broken lines. The construction is such that as the bearing component is snapped into position the flanges 54 resiliently deform and pass below the flanges 53 so that the edges of each segment 55 locate the bearing component against posterior and anterior movement.

When such a tray is to be used with a sliding meniscal component of the kind described herein and with reference to the drawings the bearing component is cut away on each side along a line indicated by reference numerals 56 and 57 so that the relative movement between the tray and the bearing component is not impaired.

It will be appreciated that with the constructions described above in which the bearing component moves in relation to the tray that the bearing components have to be handed.

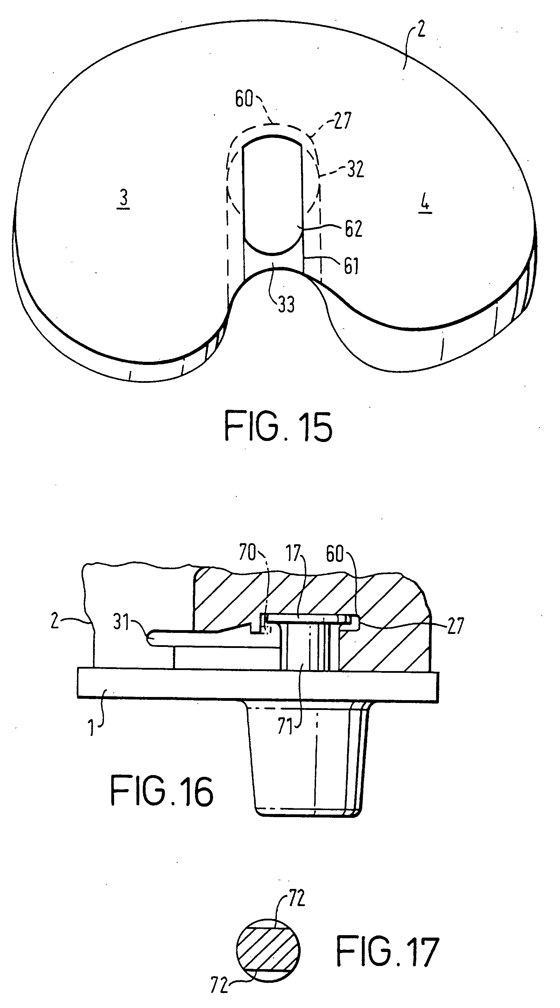

Figures 14 and 15 show a construction in which the bearing component 2 is not movable in relation to the tray 1 once it has been secured in position. The same reference numerals are used to indicate similar parts to those described with regard to the preceding constructions.

In this fixed arrangement the upper groove 27 is considerably shortened so that its anterior end 60 extends merely as an extended groove surrounding the upper end of the well 32. Thus, the well now has a surrounding groove at each side and at its anterior side, this groove terminating at each side in the wall 33.

The curved track 22 is replaced by a straight track 61 and a straight recess 62. The securing flange 24 of the previous constructions is, of course, now straight and is indicated by reference numeral 63.

With this construction the bearing component 2 is fitted in a similar way to that described with regard to Figures 6 to 8 but the shortened upper groove 27 ensures that the position on the tray 1 is fixed. Moreover, due to the straight guide track 61 there is no sideways movement, the abutment 16 acting posteriorly of the boss 8 against the sides of the straight securing flange 63.

With this arrangement the bearing component 2 can be removed if desired in a similar manner to that described with regard to the removal of the bearing component in the construction shown in Figures 1 to 11.

If it is intended that the bearing component should be fixed and non-removable then a projecting lip can be provided on the abutment 16 to engage a co-operating lip 48 on the wall 33 in a similar manner to that described with regard to Figure 12.

Figures 16 and 17 show an alternative construction for use when there is no requirement for relative movement between the bearing component and the tray 1. The same reference numerals are used to indicate similar parts to those described with regard to the other Figures. In this arrangement a straight track 61 and straight recess 62 are again employed and the anterior end 60 of the groove is again shortened and is similar to the construction shown in Figures 14 and 15. The posterior end of the upper groove 27 is also shortened so that the upper groove is effectively circular and the wall 33 is replaced by a deformable retaining wall 70.

The abutment 16 is deleted from the tray 1 and the boss 71 is provided with flats 72 on its opposed sides. This portion of the boss 71 could be replaced by a collar carrying the flats and which, when the screw 7 was tightly engaged into the stem, hold the collar in a fixed posterior anterior position.

The bearing component 2 is again loaded into a position in a similar manner to that described with regard to the preceding constructions but once it is in position in the well 32 the flats on its sides engage the sides of the track 61 and prevent rotation of the bearing component. The location of the flange 17 in the modified groove 27 prevents upward removal of the bearing component and due to the groove 27 being substantially circular movement in all directions is prevented.

The bearing component 2 can be removed in a similar manner to the constructions described above. If however it is intended that the bearing component should be unremovable then at the lower end of the wall 70 a lip, similar to the lip 48 shown in Figure 12, can be provided which engages beneath the flange 17 preventing removal.

In all the constructions shown in Figures 14 to 16 the tray 1 and bearing component 2 can also be provided with medial and lateral retaining means as described with regard to Figure 13.

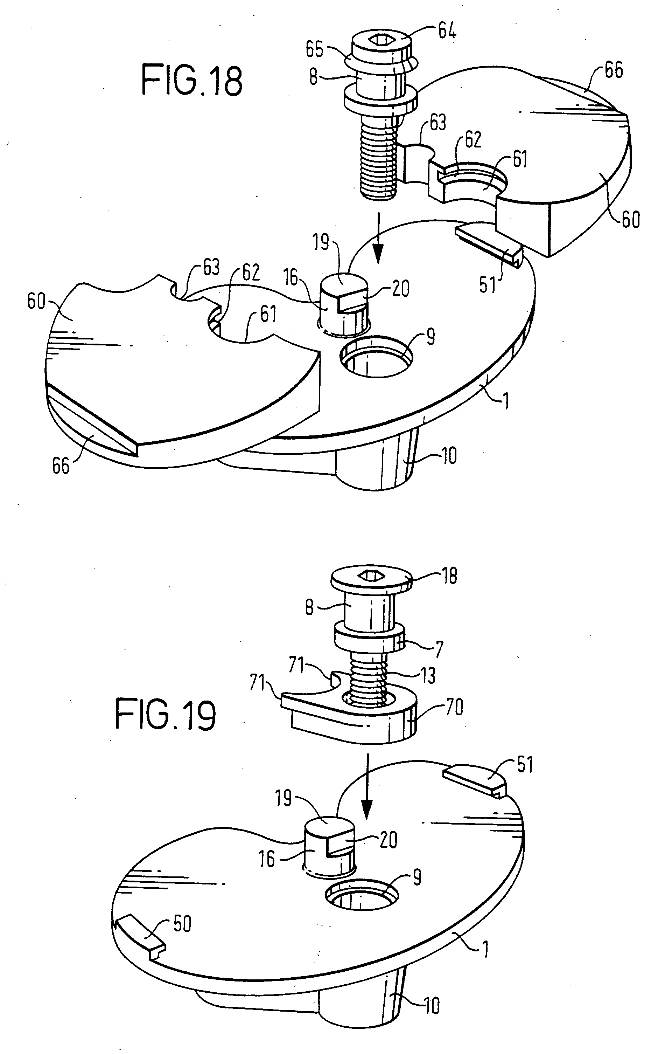

Figure 18 shows an alternative construction in which two bearing components are employed. With this arrangement the tray has an upper configuration similar to that shown in Figure 13 of the previous embodiments, that is it is provided with abutments 50 and 51. Each of the bearing components 60 is provided with a re-entrant portion 61 which carries a groove 62. A second re-entrant portion 63 is also provided to encompass the abutment 16 when the bearing component is in position.

A screw 64 acts to hold the stem 11 (not shown in Figure 18) in place and this screw 64 is formed with a boss shaped head 8 above which is a projecting tapered flange 65.

With the screw 64 in place outwardly projecting flanges 66 provided on the bearing components are first pushed into place beneath the abutments 50, 51 and are then snapped downwards so that the grooves 62 snap into place beneath the tapered flange 65. The bearing components are further located by the abutment 16.

In order to remove the bearing components it is merely necessary to insert a spatula beneath the components and between them and the tray 1 and to lever upwards so that the groove 62 snaps out of position on the tapered flange 65.

Figure 19 shows another alternative construction in which a screw 7 similar to that used in previous embodiments is employed but in this case the screw locates a location member 70 which is undercut on its lower side to provide projecting flanges 71 on each side. The general construction of the tray 1 is similar to that described with regard to Figure 18 and two bearing components 60, again similar to that shown in Figure 18, can be used. In this construction however the ends of the bearing components are not provided with the re-entrant portions 61 but have a straight corner edge which snaps under the appropriate flange 71 on the retaining member 70.

In all the above constructions the bearing components can be made of any suitable material for example ultra high molecular weight polyethylene and the tray and stem can be formed from, for example, stainless steel.

## Claims

1. A tibial element for a replacement knee prosthesis comprising a tibial tray (1), fastening means (7,8,9) for securing an attachment element (11) to the lower part of said tray (1), **characterised in that** the upper end of said fastening means (7,8,9) is also provided with independently operable securing means (8,17) which engage a co-operating construction (22,24) on a bearing component (2) to secure the bearing component (2) to the upper part of the tibial tray (1).

2. A tibial element as claimed in claim 1 **characterised in that** said independently operable securing means (8,17) are releasable.

3. A tibial element as claimed in claim 1 **characterised in that** said independently operable securing means (8,17) are non-releasable.

4. A tibial element as claimed in claim 1, claim 2 or claim 3 **characterised in that** said bearing component (2) can slide in relation to the tray (1) when secured.

5. A tibial element as claimed in claim 4 **characterised in that** said securing means (8,17) forms part of control means acting between the tray (1) and said bearing component (2) for controlling sliding movement of the bearing component (2) in relation to the tray.

6. A tibial element as claimed in claim 5 **characterised in that** said control means includes a curved track (22) in the base of the bearing component (2).

7. A tibial element as claimed in claim 6 **characterised in that** said control means also includes guide means (8,16) which incorporate a guide (8) formed by said securing means (8,17).

8. A tibial element as claimed in claim 1, claim 2 or claim 3 **characterised in that** said bearing component (2) is fixed in position in relation to the tray (1) when secured.

9. A tibial element as claimed in any one of the preceding claims **characterised in that** said fastening means (7,8,9) is provided with a boss (8) at its upper end which is shaped to co-operate with the co-operating retaining construction (20,24) on the bearing component (2).

10. A tibial element as claimed in claim 9 **characterised in that** said boss (8) has a projecting flange (17) shaped to engage a securing flange (24) provided on the bearing component (2).

11. A tibial element as claimed in claim 10 **characterised in that** two vertically spaced apart groves (26,27) are provided in the bearing component (2) the upper groove (26) providing the securing flange (24) and the grooves being interconnected to allow the projecting flange (17) on said boss (8) to move between them.

12. A tibial element as claimed in any one of preceding claims 1, 2, 3, 8, 9, 10 or 11 **characterised in that** two bearing components (60) are provided, each component (60) having a co-operating construction (61,62) which engages the independently operable securing means (65).

13. A tibial element as claimed in claim 12 **characterised in that** each bearing component (60) is independently engageable with the securing means (65).

14. A tibial element as claimed in any one of the preceding claims **characterised in that** the tray (1) is standard for both left and right knees and the bearing components (2,60) are handed.

15. A tibial element as claimed in claim 15 **characterised in that** the tray (1) is substantially symmetrical about a vertical axis.

## Patentansprüche

1. Tibiateil für eine Knieersatzprothese, umfassend ein Tibiaplateau (1), eine Befestigungseinrichtung (7,8,9) zum Befestigen eines Zubehörteils (11) am unteren Teil des Plateaus (1), **dadurch gekennzeichnet, dass** das obere Ende der Befestigungseinrichtung (7,8,9) auch mit unabhängig betätigbaren Sicherungseinrichtungen (8,17) versehen ist, die mit einer damit zusammenwirkenden Konstruktion (22,24) auf einer Lagerkomponente (2) in Eingriff treten, um die Lagerkomponente (2) am oberen Teil des Tibiaplateaus (1) zu sichern.

2. Tibiateil nach Anspruch 1, **dadurch gekennzeichnet, dass** die unabhängig betätigbaren Sicherungseinrichtungen (8,17) lösbar sind.

3. Tibiateil nach Anspruch 1, **dadurch gekennzeichnet, dass** die unabhängig betätigbaren Sicherungseinrichtungen (8,17) nicht lösbar sind.

4. Tibiateil nach Anspruch 1, Anspruch 2 oder Anspruch 3, **dadurch gekennzeichnet, dass** sich die Lagerkomponente (2) in Bezug zum Plateau (1) verschieben kann, wenn sie gesichert ist.

5. Tibiateil nach Anspruch 4, **dadurch gekennzeichnet, dass** die sicherungseinrichtung (8,17) einen Teil einer zwischen dem Plateau (1) und der Lagerkomponente (2) wirkenden Steuereinrichtung zur Steuerung einer Verschiebebewegung der Lagerkomponente (2) in Bezug zum Plateau bildet.

6. Tibiateil nach Anspruch 5, **dadurch gekennzeichnet, dass** die Steuereinrichtung eine gekrümmte Bewegungsbahn (22) im Fuß der Lagerkomponente (2) einschließt.

7. Tibiateil nach Anspruch 6, **dadurch gekennzeichnet, dass** die Steuereinrichtung auch Führungseinrichtungen (8,16) einschließt, die eine von den Sicherungseinrichtungen (8,17) gebildete Führung (8) einschließen.

8. Tibiateil nach Anspruch 1, Anspruch 2 oder Anspruch 3, **dadurch gekennzeichnet, dass** die Lagerkomponente (2) in Bezug zum Plateau (1) in ihrer Position fixiert ist, wenn sie gesichert ist.

9. Tibiateil nach einem der vorangehenden Ansprüche, **dadurch gekennzeichnet, dass** die Befestigungseinrichtung (7,8,9) mit einem Knauf (8) an ihrem oberen Ende versehen ist, der geformt ist, um mit der damit zusammenwirkenden Haltekonstruktion (20,24) auf der Lagerkomponente (2) zusammenzuwirken.

10. Tibiateil nach Anspruch 9, **dadurch gekennzeichnet, dass** der Knauf (8) einen überstehenden Flansch (17) aufweist, der geformt ist, um mit einem auf der Lagerkomponente (2) vorgesehenen Sicherungsflansch (24) in Eingriff zu treten.

11. Tibiateil nach Anspruch 10, **dadurch gekennzeichnet, dass** zwei in vertikalem Abstand angeordnete Nuten (26,27) in der Lagerkomponente (2) vorgesehen sind, wobei die obere Nut (26) den Sicherungsflansch (24) liefert, und die Nuten untereinander verbunden sind, um es zuzulassen, dass sich der überstehende Flansch (17) auf dem Knauf (8) zwischen ihnen bewegt.

12. Tibiateil nach einem der vorangehenden Ansprüche 1, 2, 3, 8, 9, 10 oder 11, **dadurch gekennzeichnet, dass** zwei Lagerkomponenten (60) vorgesehen sind, wobei jede Komponente (60) eine zusammenwirkende Konstruktion (61,62) aufweist, die mit den unabhängig betätigbaren Sicherungseinrichtungen (65) in Eingriff tritt.

13. Tibiateil nach Anspruch 12, **dadurch gekennzeichnet, dass** jede Lagerkomponente (60) unabhängig mit den Sicherungseinrichtung (65) in Eingriff bringbar ist.

14. Tibiateil nach einem der vorangehenden Ansprüche, **dadurch gekennzeichnet, dass** das Plateau (1) für sowohl das linke und rechte Knie einheitlich ist und die Lagerkomponenten (2,60) händig sind.

15. Tibiateil nach Anspruch 14, **dadurch gekennzeichnet, dass** das Plateau (1) im Wesentlichen symmetrisch um eine vertikale Achse ist.

## Revendications

1. Elément tibial pour une prothèse de genou de remplacement comprenant un plateau tibial (1), des moyens de fixation (7, 8, 9) pour fixer un élément de fixation (11) à la partie inférieure dudit plateau (1), **caractérisé en ce que** l'extrémité supérieure desdits moyens de fixation (7, 8, 9) comporte également des moyens de fixation pouvant être actionnés indépendamment (8, 17) qui s'engagent avec une construction coopérante (22, 24) sur un composant de support (2) de façon à fixer le composant de support (2) à la partie supérieure du plateau tibial (1).

2. Elément tibial selon la revendication 1, **caractérisé en ce que** lesdits moyens de fixation pouvant être actionnés indépendamment (8, 17) sont libérables.

3. Elément tibial selon la revendication 1, **caractérisé en ce que** lesdits moyens de fixation pouvant être actionnés indépendamment (8, 17) sont non-libérables.

4. Elément tibial selon la revendication 1, la revendication 2 ou la revendication 3, **caractérisé en ce que** ledit composant de support (2) peut coulisser par rapport au plateau (1) lorsqu'il est fixé.

5. Elément tibial selon la revendication 4, **caractérisé en ce que** lesdits moyens de fixation (8, 17) forment une partie de moyens de commande agissant entre le plateau (1) et ledit composant de support (2) pour commander le mouvement de coulissement du composant de support (2) par rapport au plateau.

6. Elément tibial selon la revendication 5, **caractérisé en ce que** lesdits moyens de commande comprennent une piste incurvée (22) dans la base du composant de support (2).

7. Elément tibial selon la revendication 6, **caractérisé en ce que** lesdits moyens de commande comprennent également des moyens de guidage (8, 16) qui incorporent un guide (8) formé par lesdits moyens de fixation (8, 17).

8. Elément tibial selon la revendication 1, la revendication 2 ou la revendication 3, **caractérisé en ce que** ledit composant de support (2) est fixe en position par rapport au plateau (1) lorsqu'il est fixé.

9. Elément tibial selon l'une quelconque des revendications précédentes, **caractérisé en ce que** lesdits moyens de fixation (7, 8, 9) comportent une protubérance (8) à leur extrémité supérieure, celle-ci étant mise en forme de façon à coopérer avec la construction de maintien coopérante (20, 24) sur le composant de support (2).

10. Elément tibial selon la revendication 9, **caractérisé en ce que** ladite protubérance (8) comporte un flasque saillant (17) mis en forme de façon à s'engager avec un flasque de fixation (24) disposé sur le composant de support (2).

11. Elément tibial selon la revendication 10, **caractérisé en ce que** deux rainures verticalement espacées l'une de l'autre (26, 27) sont présentes dans le composant de support (2), la rainure supérieure (26) constituant le flasque de fixation (24), et les rainures étant interconnectées de façon à permettre au flasque saillant (17) sur ladite protubérance (8) de se déplacer entre celles-ci.

12. Elément tibial selon l'une quelconque des revendications 1, 2, 3, 8, 9, 10 ou 11 qui précèdent, **caractérisé en ce que** deux composants de support (60) sont présents, chaque composant (60) ayant une construction coopérante (61, 62) qui s'engage avec les moyens de fixation pouvant être actionnés de façon indépendante (65).

13. Elément tibial selon la revendication 12, **caractérisé en ce que** chaque composant de support (60) peut s'engager de façon indépendante avec les moyens de fixation (65).

14. Elément tibial selon l'une quelconque des revendications précédentes, **caractérisé en ce que** le plateau (1) est standard pour les deux genoux gauche et droit et **en ce que** les composants de support (2, 60) sont latéralisés.

15. Elément tibial selon la revendication 14, **caractérisé en ce que** le plateau (1) est sensiblement symétrique autour d'un axe vertical.
